# EUROPEAN PATENT SPECIFICATION

(11) **EP 4 181 861 B1**
(45) Date of publication and mention of the grant of the patent: **24.01.2024**
(21) Application number: 21737473.5
(22) Date of filing: 01.07.2021
(51) Int. Cl.: C09K 3/30, A61K 8/19, A61K 8/31, A61K 8/34, A61K 8/37, A61Q 5/02, B65D 83/14

(54) **AEROSOL PRODUCT**
AEROSOLPRODUKT
PRODUIT AÉROSOL

(30) Priority: 17.07.2020 EP 20186526
(43) Date of publication of application: 24.05.2023
(73) Proprietor: Unilever IP Holdings B.V., 3013 AL Rotterdam (NL); Unilever Global IP Limited, Wirral, Merseyside CH62 4ZD (GB)
(72) Inventor: BROWN, Gillian, Wirral Merseyside CH63 3JW (GB); DAWSON, Robert, Wayne, Wirral Merseyside CH63 3JW (GB); JOINSON, Leslie, Joseph, Luke, Wirral Merseyside CH63 3JW (GB)
(74) Representative: Mathai, Neenu Grace
(86) International application number: PCT/EP2021/068168
(87) International publication number: WO 2022/012947

(56) References cited:
- EP-A1- 0 753 561
- EP-A2- 2 570 110
- WO-A1-94/01511
- US-A1- 2012 034 173
- US-B1- 6 581 807
- US-B1- 8 191 739

## Description

### Field of the Invention

The present invention relates to an aerosol product having reduced volatile organic compound (VOC) content.

### Background of the Invention

Aerosol products have been popular since their inception, because of their marked ease of use in a variety of applications. The term "aerosol" includes products that can be dispensed in a stream, spray, powder, gel or a foam. Innovations in this area of technology have provided aerosol products that contain organic solvents or water or combinations thereof, as well as products that foam upon ejection and products that delay foaming after ejection.

Typical aerosol hairspray products comprise a pressure-resistant container, a nozzle, a propellant, and a hair care formulation. A hairspray composition is normally ejected from such products via aerosol-forming nozzle. See, for example, US2009/0104138A1.

Typically, two types of propellants are used in aerosol products: liquefied gases and compressed gases.

The use of a compressed gas alone as a propellant has drawbacks, in certain applications, where the pressure is not sustained over the use of the aerosol container; the contents are over-pressurized at the beginning of the consumer's use and become under-pressurized prior to the complete use of the product. The amount of compressed gas retained in a product is dependent on its solubility in the product being dispensed. The less soluble the compressed gas is in the product, the more compressed gas is retained in the vapor phase (i.e., the headspace) within the aerosol container. Thus, internal vapour pressure of the aerosol dispenser diminishes as the contents are depleted, causing changes in the rate and characteristics of the spray. In particular, the use of nitrogen, whilst environmentally desired, as a compressed gas propellant has been discouraged in the art because of nitrogen's insolubility in the product; this insolubility causes rapid pressure depletion and changed spray characteristics, and for these reasons nitrogen generally has limited applications as a compressed gas propellant in the aerosol industry.

The addition of LPGs and/or HFCs contributes to the aerosol product's desirable spray characteristics. More specifically, certain types of liquefied gases allow the product to foam upon dispensing. However, the use of LPGs or HFCs alone in typical formulations known in the art has its own drawbacks concerning cost and environmental profile, as discussed below. Despite these drawbacks, liquefied gases are presently used as propellants in many aerosol products because of the desirability of foaming aerosol products. In order to foam upon dispensing, aerosol products must contain liquefied gas; for this reason, liquefied gas-whether LPGs, HFCs, and blends thereof-is used in the aerosol industry as the propellant in a variety of foaming aerosol products.

As mentioned above, certain concerns influence the use of LPGs. First, the use of LPGs has significant environmental concerns, as LPGs fall within a class of chemicals known as volatile organic compounds ("VOCs"). VOCs are precursors of ground level smog, which is a significant daily environmental hazard in many urbanized areas. On an individual level, VOCs have been associated with a variety of health problems, ranging from irritation to chronic problems.

Environmental sustainability has become a matter of growing interest in aerosol applications. The environment is benefited by a product that, compared to conventional aerosols, emits less propellant (e.g., volatile organic compound (VOC).

For the above reasons, there is a desire to use as little liquefied gas as possible in an aerosol product but to retain the spray characteristics.

US 8,191,739 discloses an aerosol dispenser that is pressurized by both compressed gas and liquefied gas that is able to retain desired spray characteristics, such as foaming, while reducing volatile organic compound content or hydrofluorocarbon content. US 8,191,739 does not disclose any specific spray characteristics other than foaming.

Despite the prior art, there remains a need for an aerosol product with reduced volatile organic compound while retaining the spray characteristics such as spray consistency throughout the use of the product.

It is therefore an object of the present invention to provide an aerosol product with reduced volatile organic compound while retaining the spray consistency throughout the use of the product.

Surprisingly, it has been found that spray consistency can be achieved by mixing 5 to 15% of one or more liquefied gasses by weight of the base formulation with an insoluble compressed gas, in combination with a specific inert gas fill pressure in the container.

### Summary of the Invention

Accordingly, in a first aspect, the present invention provides an aerosol product, which comprises a pressurisable container; a base formulation; and a propellant; wherein the propellant is an insoluble compressed gas mixed with 5 to 15% of one or more liquefied gasses by weight of the base formulation and wherein the pressure in the container is from 10 ×10⁵ to 11 ×10⁵ Pa (10 to 11 bar).

The term "aerosol" includes products that can be dispensed in a stream, spray, powder, gel or a foam.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed Description of the Invention

In a first aspect, the present invention relates to an aerosol product, which comprises a pressurisable container, a base formulation and a propellant.

The product comprises from 75% to 97% base formulation, preferably at least 80%, more preferably at least 85%, still more preferably at least 87% but typically not more than 95%, more preferably not more than 93% or still more preferably not more than 91% or even 90%, by total weight of the combined base formulation and propellant.

The product comprises from 3% to 25% propellant, preferably at least 5%, more preferably at least 7%, still more preferably at least 9% or even at least 10%, but typically not more than 20%, more preferably not more than 15%, even more preferably not more than 13% propellant, by total weight of the combined base formulation and propellant.

The pressurisable container comprises a container wall which encloses a reservoir for storing a base formulation, and a propellant.

The container wall may be predominantly straight, curved or tapered. The container is typically of cylindrical shape with a constant or varying circular, oval, egg shaped, or similar planar cross section.

The container preferably has a volume (so-called over fill capacity) of from 50 to 800 ml, more preferably from 200 to 600 ml, most preferably from 300 to 400 ml.

The container preferably has a diameter of from 35 to 70 mm, more preferably from 40 to 60 mm, even more preferably 45 to 55 mm.

Attached to the container is a spraying device for spraying the base formulation onto a substrate, which comprises a valve and an actuator.

The aerosol product according to the invention is preferably intended for application on human skin including hair.

The base formulation according to the invention is preferably a hair care formulation, deodorant formulation or skin formulation. Preferably, the base formulation is a hair care formulation such as hairstyling or dry shampoo formulations.

The base formulation is preferably present in amount of 40 to 100g, preferably 40 to 80 g, more preferably 40 to 60 g.

Dry shampoo formulations may comprise starch and a conditioning agent in an alcoholic composition.

The optional starch according to the invention includes starch derivates. Preferably the starch is selected from corn starch, tapioca starch, rice starch, and modified starch in particular, modified corn starch and tapioca starch. A preferred modified starch is aluminium starch octenyl succinate.

The optional starch is present in a concentration of 3 to 15%, preferably at least 6%, more preferably at least 7%, still more preferably at least 8%, even more preferably at least 9%, but typically not more than 14%, preferably not more than 13%, more preferably not more than 12%, still more preferably not more than 11%, even more preferably not more than 10% or even 9% by total weight of the combined dry shampoo formulation and propellant.

Isopropyl myristate may be used in the present invention as a preferable conditioning agent. The isopropyl myristate is optionally present in a concentration of 0.5 to 4%, preferably at least 0.75%, more preferably at least 1%, still more preferably at least 1.25%, even more preferably at least 1.5%, but typically not more than 3.5%, preferably not more than 3%, more preferably not more than 2.5%, still more preferably not more than 2%, by total weight of the combined dry shampoo formulation and propellant.

Preferably, the dry shampoo formulation comprises an anti-caking agent which is preferably silica. Preferably, the anti-caking agent is present in the base from 0.1 to 1% by total weight of the combined dry shampoo formulation and propellant.

Preferably, the dry shampoo formulation comprises an alcohol. The alcohol is preferably selected from ethanol, isopropanol, butanol, most preferably ethanol.

The alcohol is optionally present in an amount of from 50 to 90 wt % by total weight of the combined dry shampoo formulation and propellant, preferably at least 60 wt %, more preferably at least 65%, still more preferably at least 70%, even more preferably at least 75% but typically not more than 85%, more preferably 80% by total weight of the combined dry shampoo formulation and propellant.

Further optional ingredients present in the base may include propylene glycol, fragrance, emotives.

Hairstyling formulation comprises a hair styling polymer and a neutraliser in an alcoholic composition.

The hairstyling polymer is present in an amount of from 1.0 to 10 wt %, preferably from 1.0 to 8.0 wt %, most preferably from 2 to 7 wt % by total weight of the combined hair styling formulation and propellant.

The hairstyling polymer may be selected from a VA/Crotonates/Vinyl Neodecanoate Copolymer, Polyurethane-14 (and) AMP-Acrylates Copolymer (for example, DynamX available from AkzoNobel), Butyl Ester of PVM/MA Copolymer (for example, Gantrez available from Ashland), Polyester-5, Octylacrylamide/Acrylates/Butylaminoethyl Methacrylate Copolymer, imidized isobutylene/maleic anhydride copolymer (for example, Aquaflex^{™} FX-64 available from Ashland), Polyvinylpyrrolidone, Acrylates/Hydroxyesters Acrylates Copolymer (for example, Acudyne 180 available from DOW), Polyurethane-6, Acrylates/Octylacrylamide Copolymer and Acrylates Copolymer (for example, Balance CR available from AkzoNobel and Luvimer 100 P available from BASF), and more preferably from VA/Crotonates/Vinyl Neodecanoate Copolymer (for example, Resyn 28-2930 available from AkzoNobel) and Octylacrylamide/Acrylates/Butylaminoethyl Methacrylate Copolymer (for example, Amphomer available from AkzoNobel).

Preferably the styling resin comprises VA/Crotonates/Vinyl Neodecanoate Copolymer (for example, Resyn 28-2930 available from AkzoNobel) and Octylacrylamide/Acrylates/Butylaminoethyl Methacrylate Copolymer (for example, Amphomer available from AkzoNobel).

The neutralizer provides an alkaline base. The neutralizer is preferably selected from potassium hydroxide, sodium hydroxide, triisopropanolamine, 2-aminobutanol, 2-aminomethyl propanol, aminoethylpropandiol, dimethyl stearamine, sodium silicate, tetrahydroxypropyl ethylenediamine, ammonia, triethanolamine, trimethylamine, aminomerhylpropandiol, and more preferably 2-aminomethyl propanol, dimethyl stearamine and 2-aminobutanol and mixtures thereof.

Preferably, the hair styling formulation comprises an alcohol. The optional alcohol is preferably selected from ethanol, isopropanol, butanol, most preferably ethanol.

The alcohol is optionally present in an amount of from 30 to 75 wt % by total weight of the hairstyling formulation and propellant, preferably from 35 to 70 wt %, more preferably from 35 to 60 wt %, most preferably from 35 to 55 wt %, by total weight of the combined hairstyling formulation and propellant.

The formulation may also comprise other adjuncts commonly used in hair sprays. Preferably perfume, silicone (for example phenyl trimethicone, cyclopentylsiloxane, PEG/PPG-20/15 Dimethicone), emotives, corrosion inhibitors, emollients, UV absorbers (for example benzopheonone-4, ethyl hexyl methoxy cinnamate) and mixtures thereof.

### Propellant

The propellant used in the present invention is an insoluble compressed gas mixed with 5 to 15% of one or more liquefied gasses by weight of the base formulation.

Suitable compressed gases should be inert and insoluble. In a preferred embodiment, nitrogen is the compressed gas, because nitrogen is inert and is not soluble in water. Other inert, insoluble compressed gases include helium and argon; both of these gases would be expected to work effectively as compressed gas propellants. Compared to nitrogen, helium and argon present practical drawbacks because they are more expensive and impractical than nitrogen for use in aerosol products. However, from a scientific standpoint, helium and argon would be expected to be acceptable propellants, due to their inert nature and insolubility. Compared to argon, helium would be a better substitute for nitrogen, although helium's low molecular weight may cause significant leakage problems in an aerosol container. In the aerosol industry, carbon dioxide and nitrous oxide have also been used as compressed gases to act as propellants. However, nitrous oxide and carbon dioxide are soluble in water and a variety of organic solvents, and to the extent the compressed gas is soluble, the vapor pressure of the compressed gas will be depressed due to its solubility in the aerosol formula concentrate. Hence, insoluble compressed gasses are used in the present invention with nitrogen being the most preferred.

Without wishing to be bound by a theory, it is found that inclusion of one or more liquified gasses at levels of 5 to 15%, preferably 6 to 14% by weight of the base to the compressed gas counteracts the pressure drop observed towards the end of the can lifetime.

When filling with the propellant, after adding the base formulation, the container is first filled with 3 to 9g, preferably 4 to 8g of liquefied gas followed by the compressed gas to reach an inert gas fill pressure of 9 ×10⁵ to 11 ×10⁵ Pa (9 to 11 bar), preferably 10 ×10⁵ to 11 ×10⁵ Pa (10 to 11 bar), most preferably 10.7×10⁵ Pa (10.7 bar).

The compressed gas is added to the container in an amount sufficient to provide an absolute inert gas fill pressure of 10×10⁵ to 11×10⁵ Pa (10 to 11 bar), most preferably 10.7×10⁵ Pa (10.7 bar).

Liquefied gases that are used as propellants can include liquefied petroleum gases ("LPGs") and hydrofluorocarbons ("HFCs"). As used in the context of this invention, the term "liquefied gas" is used to encompass both LPGs and HFCs. In the aerosol industry, LPGs include hydrocarbon propellants (e.g., propane, n-butane, isobutane). In the aerosol industry, HFCs include 1,1 difluoroethane (CH3CHF2), known in the aerosol industry as "152a," and 1,1,1,2 tetrafluoroethane (CF3CH2F), known in the aerosol industry as "134a."

Three LPGs are preferred: n-butane, isobutane, and propane. These LPGs fall into a class also often referred to as "hydrocarbon propellants." N-butane (C4H10(n)) is commonly referred to as "A-17" in the aerosol industry, as it has a 70°F (21°C) vapor pressure of 17 psig (1.17 bar(g), 1.17×10⁵ Pa(g)). Isobutane (C4H10(iso)) is commonly referred to as "A-31" in the aerosol industry, as it has a 70°F (21°C) vapor pressure of 31 psig (2.14 bar(g), 2.14×10⁵ Pa(g)). Propane (C3H8) is commonly referred to as "A-108" in the aerosol industry, as it has a 70°F (21°C) vapor pressure of 108 psig (7.45 bar(g), 7.45×10⁵ Pa(g)). Most preferred LPG is a blend of n-butane, isobutane, and propane.

In addition to n-butane, isobutane, and propane, other hydrocarbon propellants may also be used. For example, isopentane and n-pentane may also behave in the same manner as n-butane, isobutane, and propane.

The invention will now be further described by reference to the following Examples. In the Examples, all percentages are by weight based on total weight, unless otherwise specified.

### Exam ples

### Example 1: Effect of the invention on pressure drop

Dry shampoos using nitrogen mixed with varying levels of LPG as propellant were assessed for pressure drop.

An assessment of pressure was performed using a Digital pressure gauge over the can lifetime. Each hairspray product was assessed at the start with weight and pressure recorded, using the confirmed fill weight of the product, the weight at each quartile was calculated. The products were then sprayed continuously until the weight met the calculated quartile weight, where the pressure was remeasured.

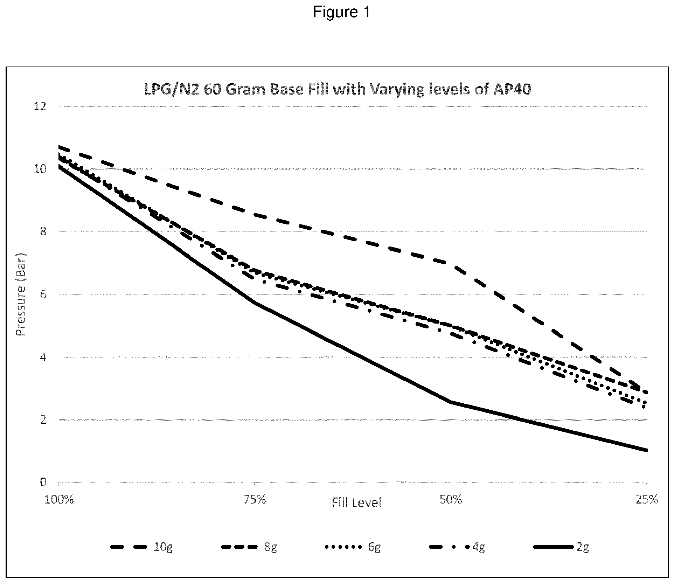

Results are given in Figure 1.

When the aerosol product is used, a small amount of propellant is removed during dispense and there is an increase in the volume of the container headspace, reducing the pressure in the container. The resulting lower pressure created within the container enables the evaporation of liquid LPG to gaseous LPG to increase the pressure inside the container back to an equilibrium. This is called rebalancing. The graph in Figure 1 shows that addition of 2g LPG (3.3%) has a minimal effect on the maintenance of the pressure due to rebalancing. At 10g LPG addition (16.7%), the pressure is maintained well at when the container is more than half full, but drops quickly after that. In the range of 5-15% (4-8 grams) the pressure drop is gradual and steady, providing adequate pressure rebalancing, without wastage of the propellant and resulting in consistent spray properties.

## Claims

1. An aerosol product, which comprises
a a pressurisable container;
b a base formulation; and
c a propellant;
wherein the propellant is an insoluble compressed gas mixed with 5 to 15% of one or more liquefied gasses by weight of the base formulation and wherein the pressure in the container is from 10×10⁵ to 11×10⁵ Pa (10 to 11 bar).

2. An aerosol product according to claim 1 wherein the insoluble compressed gas is nitrogen.

3. An aerosol product according to any one of the preceding claims wherein the compressed gas is mixed with 6 to 14% of liquefied gas by weight of the base formulation.

4. An aerosol product according to any one of the preceding claims wherein the base formulation is a hair care formulation.

5. An aerosol product according to any one of the preceding claims wherein the liquefied gas is a blend of propane, isobutane and n-butane.

## Patentansprüche

1. Aerosolprodukt, das umfasst
a einen druckbeaufschlagbaren Behälter;
b eine Basisformulierung; und
c ein Treibmittel;
wobei das Treibmittel ein unlösliches komprimiertes Gas ist, das mit 5 bis 15% eines oder mehrerer verflüssigter Gase, bezogen auf das Gewicht der Basisformulierung, gemischt ist und wobei der Druck in dem Behälter 10×10⁵ bis 11×10⁵ Pa (10 bis 11 bar) beträgt.

2. Aerosolprodukt nach Anspruch 1, wobei das unlösliche komprimierte Gas Stickstoff ist.

3. Aerosolprodukt nach irgendeinem der vorhergehenden Ansprüche, wobei das komprimierte Gas mit 6 bis 14% verflüssigtem Gas, bezogen auf das Gewicht der Basisformulierung, gemischt ist.

4. Aerosolprodukt nach irgendeinem der vorhergehenden Ansprüche, wobei die Basisformulierung eine Haarpflegeformulierung ist.

5. Aerosolprodukt nach irgendeinem der vorhergehenden Ansprüche, wobei das verflüssigte Gas eine Mischung aus Propan, Isobutan und n-Butan ist.

## Revendications

1. Produit aérosol, qui comprend :
a un récipient pouvant être mis sous pression ;
b une formulation de base ; et
c un propulseur ;
dans lequel le propulseur est un gaz comprimé insoluble mélangé avec 5 à 15 % en poids d'un ou plusieurs gaz liquéfiés de la formulation de base et dans lequel la pression dans le récipient est de la 10 × 10⁵ à 11 × 10⁵ Pa (10 à 11 bars).

2. Produit aérosol selon la revendication 1 dans lequel le gaz comprimé insoluble est de l'azote.

3. Produit aérosol selon l'une quelconque des revendications précédentes dans lequel le gaz comprimé est mélangé avec 6 à 14 % en poids de gaz liquéfié de la formulation de base.

4. Produit aérosol selon l'une quelconque des revendications précédentes dans lequel la formulation de base est une formulation de soin capillaire.

5. Produit aérosol selon l'une quelconque des revendications précédentes dans lequel le gaz liquéfié est un mélange de propane, d'isobutane et de n-butane.
